# EUROPEAN PATENT APPLICATION

(11) **EP 0 609 904 A1**
(43) Date of publication of application: **10.08.1994**
(21) Application number: 94102551.2
(22) Date of filing: 19.10.1987
(51) Int. Cl.: B29C 47/02

(54) **Method of manufacturing a flexible tube**

(30) Priority: 17.10.1986 GB 8624949; 20.07.1987 AU 3234/87
(62) Divisional of application: 87906834.4
(71) Applicant: PATTULLO, Norman, Giffnock Glasgow G46 6RH (GB)
(72) Inventor: O'Neil, Alexander George Brian, Subiaco 6008, Perth (AU)
(74) Representative: Pattullo, Norman

(57) **Abstract**

A method of manufacturing a flexible tube, comprising forming around a thin elongate member a body of flexible material so that the elongate member is embedded in the body and withdrawing the elongate member longitudinally from the body to form a bore through the body.

## Description

### METHOD

This invention relates to a method of manufacturing a flexible tube.

It is often necessary to provide a constant or intermittent flow of drugs to a patient under treatment, as for example in the case of a diabetic, and hitherto such controlled feeding of drugs has been carried out by using apparatus such as electrically-driven screw mechanisms actuated in a programmed manner by microprocessor. These apparatus, however, are expensive and require power sources that render the apparatus bulky and unwieldy.

Other systems that have previously been proposed employ resilient drug reservoirs which contain drugs under pressure, causing inflation of the reservoir to produce a positive head of liquid. This pressure head results in the drug being driven from the reservoir along a narrow-bore tube to the patient. Such systems are described for example in US Patents No 3,469,578 (Bierman) and No 3,486,539 (Jacuzzi). In the Bierman and Jacuzzi Patents the outflow rate of liquid drug from the reservoir is controlled by a porous plug through which the drug is led, the porosity of the plug determining the rate of drug flow.

Other similar arrangements have been proposed such as in US Patent No. 3,468,308 (Bierman) in which the flow rate from the reservoir is controlled either by passage through a rigid needle or through a porous mass, and UK Patent No. 1,454,310 (Alza Corporation) in which the flow is controlled by an adjustable valve or variable restriction in the outlet line from the reservoir.

US Patent No. 4,626,243 (Singh et al) provides a reservoir that deforms under applied external pressure from a pressurised gas cylinder, and the outflow of liquid from the reservoir is determined by a flow restrictor in the outlet line comprising a narrow-bore tube. The bore can be provided by a length of hypodermic needle tubing or by a short length of thick-walled elastomer tube contained within an outer metallic tube.

In all of these prior art arrangements the apparatus for controlling the outflow of liquid from the reservoir is disposed in a rigid section of liquid line between the reservoir and a discharge line which is usually in the form of flexible tubing.

It is often necessary or desirable to provide an extremely slow rate of delivery of a drug to a patient, perhaps of the order of 0.1 ml/hour, but the prior art apparatus has been able to provide only down to about 2 ml per hour. This is due to the limitations dictated by the nature of the above flow restricting devices, all of which are rigid structures inserted in the flow line; as the resistance to flow increases with increasing length of the flow restrictor, the overall flexibility of the tubing between the reservoir and the patient suffers if very slow rates of delivery are required.

According to the present invention there is provided fluid feed apparatus comprising a reservoir for fluid, means for ejecting fluid through an outlet of the reservoir, a flexible tube communicating with the outlet of the reservoir for receiving fluid therefrom, the tube comprising a flexible wall defining a bore for throughflow of fluid, the cross-sectional dimensions of the wall and the bore being such as to maintain the bore substantially uniform in cross-section during flexing of the tube.

The through bore is sufficiently small in cross-section to allow a substantial pressure difference to be maintained between its inlet and its outlet, so that a gradually reducing high pressure at its inlet maintains a constant low pressure at its outlet. The tube may be for example of silicone rubber, polyurethane or polyvinyl chloride. The wall of the conduit preferably does not allow significant expansion of the bore.

It is essential that the tube should not kink or otherwise deform to an extent that would cause the diameter of the bore to be altered in use, as this would result in uncontrolled fluid flow. This is most effectively achieved by having the ratio of the diameter of the tube to the diameter of the bore greater than 2:1, preferably 5:1, and the ratio can be very effectively around 50:1.

The bore diameter can be selected depending on the desired fluid flow characteristics, and in general it has been found that for drug infusion a bore diameter of from 0.05 to 0.25 mm can provide readily controllable and precise flow. As the resistance to flow depends on the length as well as the diameter of the bore, a number of tubes may be interconnected to provide a long flow path for the fluid if greater resistance, and therefore slower flow, is required.

The flexible nature of the tube allows such length extensions to be made without rendering the apparatus unweildy or obtrusive.

The reservoir may be in the form of a cylinder, such for example as a syringe, having a piston therein for moving fluid in the cylinder to an outlet at one end of the cylinder; the piston and cylinder may be relatively movable under the action of a spring which may be disposed internally or externally of the cylinder.

Alternatively the reservoir may be in the form of an expandible resilient bladder member which can be inflated with fluid to pressurise it, the pressure then being relieved by outflow of the fluid through the flexible tube. In order to avoid the problem of substantial loss of pressure as the bladder nears empty, which would leave a quantity of of fluid in the bladder with insufficient pressure to force it through the tube, it is of advantage to occupy such "dead space" with an insert of greater volume than the unexpanded volume of the bladder. The reservoir wall then retains a degree of resilience, so that there is always a positive pressure exerted on fluid within the reservoir. The insert should have fluid channel means to allow escape of fluid from the collapsing reservoir. The insert may have a shape which stretches the collapsing reservoir in a manner which minimises the pressure drop between the reservoir when full and the reservoir when collapsed; such an insert is preferably of elongate, narrow profile.

A further method of reducing pressure loss in the bladder is to provide a second resilient inflatable reservoir of greater volume than the bladder in communication with the bladder so that the reservoir provides fluid which replenishes the bladder as the bladder discharges fluid through the flexible tube.

The bladder may be formed by injection moulding.

Further according to the invention there is provided a method of manufacturing flexible tube for use in fluid feed apparatus, comprising forming around a thin elongate member a body of flexible material so that the elongate member is embedded in said body, and withdrawing the elongate member longitudinally from said body thereby to form a bore through the body.

The elongate member may be a wire, and the body may be formed around the wire by extrusion, by moulding or by other techniques. Extrusion is especially effective.

The invention also provides a method of feeding fluids, comprising loading a series of fluids in a predetermined order and quantity into a container, applying pressure to an inlet of the container and allowing the fluids to emerge from an outlet of the container in said predetermined order and quantity under the influence of said pressure.

The container is preferably in the form of a conduit into which are loaded the fluids to be fed. The fluids may be predetermined quantities and types of drugs for injection into a patient, the drugs being separated in the conduit by gas such as air, or by liquids immiscible with the drugs.

The apparatus of the invention may be provided in a branch line of a fluid conduit. This allows a predetermined quantity of a drug, for example, to be injected at a controlled rate into a fluid conduit to a patient without interrupting flow through the fluid conduit. A valve may be provided in the branch line for connection and disconnection of the reservoir as required, and the valve may be either upstream or downstream of the flexible tube.

The apparatus of the invention may be adapted to provide a portable system for providing a supply of drugs to a patient, by providing an injection needle at the downstream end of the apparatus and connection means for mounting the apparatus on a limb or other part of the body of the patient.

The apparatus and method of the invention may also provide a switching system or detonator for industrial application, by loading into the fluid reservoir an activating fluid preceded by a non-activating fluid, so that emergence of the activating fluid provides the switch or detonation at a predetermined time from initial actuation of the apparatus.

It will be appreciated that the apparatus of the invention can be gravity-independent in operation, and therefore adaptable to portable use and to use in weightless conditions such as in outer space. The portable aspect of the apparatus allows it to be used for emergency rapid-fluid-replacement conditions. Preferably a pressure head equivalent to 200-600 mm of mercury is derived from the reservoir.

Embodiments of the present invention will now be described with reference to the accompanying drawings in which:-
Fig. 1 is a side view of apparatus of this invention;
Fig. 2 is a side view illustrating a method of manufacture of the invention;
Fig. 3 is a side view of the drug storage reservoir shown in Fig. 1;
Fig. 4 is a sectional side view showing the fitting of the balloon of Fig. 1 onto the T-piece;
Fig. 5 is a side view of apparatus of the invention for continuous saline supply with intermittent drug supply;
Fig. 6 is a schematic side view of apparatus of the invention for maintaining a constant fluid pressure within a supply balloon;
Fig. 7 is a side view of portable apparatus of the invention;
Figs. 8 and 9 are views of alternative forms of the apparatus of the invention incorporating flushing systems;
Fig. 10 shows a fluid storage system for use with apparatus of the invention;
Fig. 11 is a sectional side elevation of a further embodiment of the apparatus of this invention;
Fig. 12 is a sectional side elevation of an alternative form of syringe to that shown in Fig. 11; and
Fig. 13 is a schematic view of an alternative form of reservoir and ejection means for fluid.

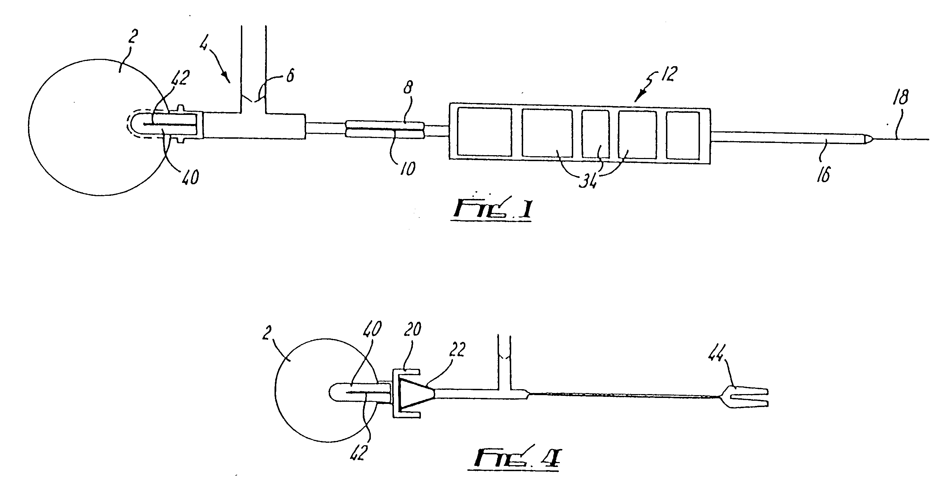

Referring to Fig. 1, the apparatus of this embodiment of the invention comprises a moulded silicone rubber bladder or balloon 2, a T-piece adaptor 4 having a one-way valve 6 connected to the leg of the T, a resistance conduit 8 of silicone rubber having a central bore 10 through it, a drug container 12 containing discrete sections or doses 34 of drugs in a predetermined order and quantity for feeding to a particular patient, tubing 16 connected to the outlet of the container 12 and a needle 18 for insertion in the patient.

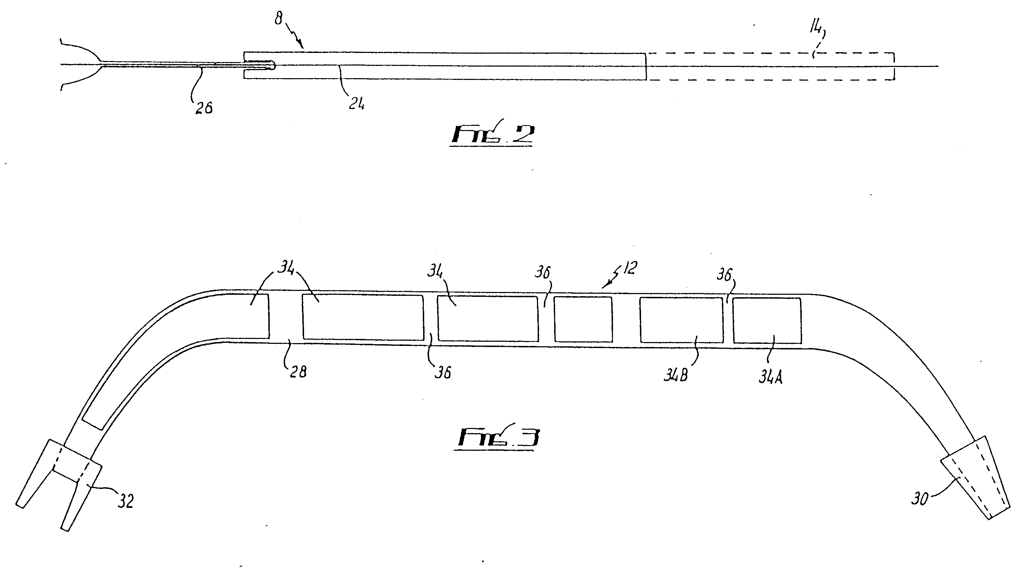

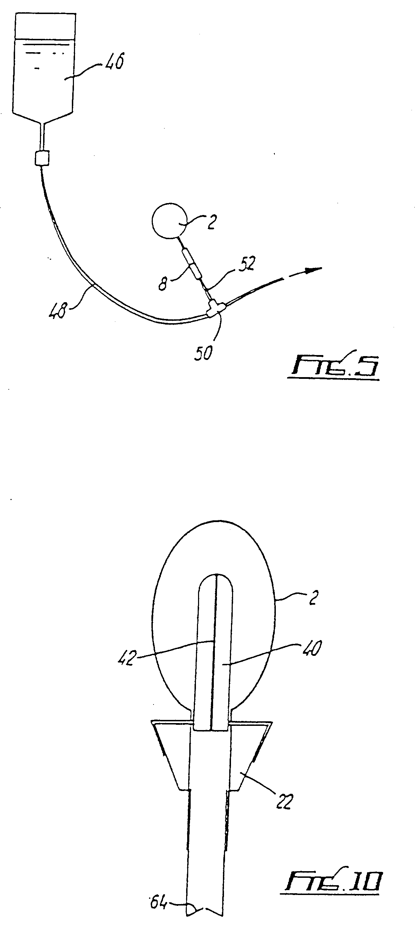

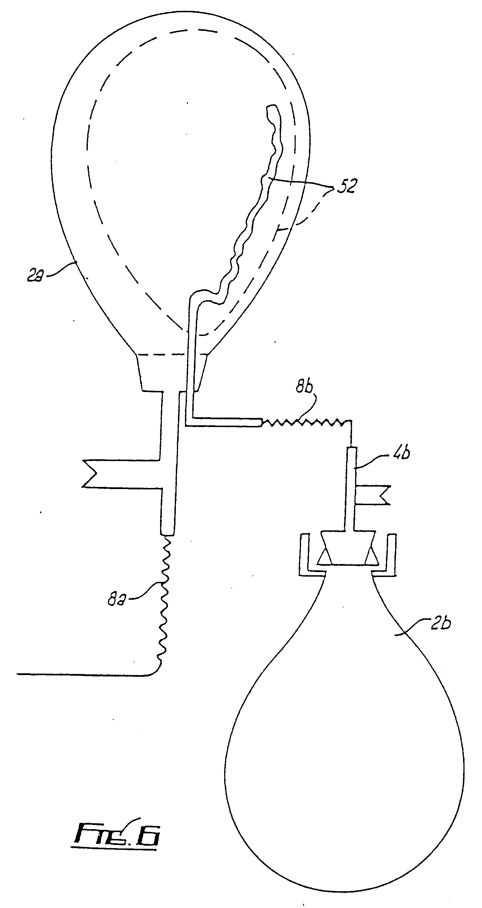

The balloon 2 is relatively thick-walled at 0.4 mm and is thereby capable of withstanding high pressure within it. The balloon is manufactured by a liquid silicone moulding technique onto a collar 20 (Fig. 4) which is 8 mm in length and is held in place on the T-piece 4 by a reverse-tapered surface 22 producing a tight fit.

A plastics insert 40 extends into the balloon 2 to limit the extent of collapse of the balloon walls on emptying. The insert 40 is selected to ensure that the collapsed balloon (shown dotted in Fig. 1) retains an element of resilience, so that a positive pressure is always maintained for fluid within the balloon 2. The insert 40 has longitudinal grooves 42 in its outer face to allow outflow of fluid along them as the balloon 2 deflates. This assists in maintaining a constant pressure of fluid from the balloon during its deflation and ensures that the balloon 2 empties itself of fluid on deflation.

The resistance conduit 8 is formed (Fig. 2) by extruding silicone rubber as a 4 mm diameter solid cylinder around a wire 24 of from 0.025 to 0.076, in the present case 0.063 mm diameter and then stripping sections 14 of the extrusion from the wire 24 to provide narrow-bore conduits of a required length of 8 cm. A needle 26 is passed around the wire 24 into the silicone rubber body, so that the wire 24 acts as a guide for the needle 26 to pass centrally through the body; without the wire the tight-fitting needle 26 tends to move off-centre as it passes along the body. After insertion of the needle 26 the wire 24 is removed.

The drug container 12 is in the form of a thin tube 28 (Fig. 3) having flexible tubing at each end terminating in male 30 and female 32 luer lock fittings. A series of drug doses 34 are inserted sequentially within the tube 28 as shown, each dose 34 being separated from adjacent doses by an air gap 36, although in other embodiments the air 36 can be replaced by a liquid such as an oil which is immiscible with the drugs.

The drug doses 34 are selected to be appropriate to the needs of a particular patient over a period of time, and are packaged within the tube 28 so as to travel along it without mixing with adjacent doses. The concentrations and quantities of the doses are also selected to compensate for the gradual reduction in pressure of the balloon 2 as it empties.

In use, the balloon 2 is inflated to a pressure of 300 mm of mercury by introduction of water through the one-way valve 6, so that the balloon 2 acts as a supply of high-pressure fluid to the resistance conduit 8 through the T-piece adaptor 4.

The small bore of the conduit 8 ensures slow leakage of water through it at a rate of 1 ml per hour, under the driving pressure provided by the balloon 2. This flow enters the drug container 12 and pushes the drug doses 34 slowly towards the outlet of the container 12 and sequentially along the outlet tubing into the needle 18 which is inserted into the patient. Each dose 34 is thereby delivered to the patient subcutaneously or intravenously in a controlled manner and at a predetermined rate.

Each dose 34 is selected and positioned initially in the container 12 as prescribed by the doctor so that, for example, the first dose 34A is of low strength while the second 34B is of higher strength. With the knowledge of the dose's flow rate into the patient, as determined by the resistance conduit 8, the duration of each dose feed can be easily predicted with certainty.

Considerable advantages accrue from this embodiment of the invention. For example, diabetics or other patients can be fed a preprogrammed insulin or other drug regime in disposable low-cost device which requires no electrical motors or controllers; the bore of the resistance conduit 8 can be selected to give a known rate of feed which remains constant over a period of time; the drug doses 34 can be selected and positioned in the container 12 to give continuous or intermittent injection of the drugs, and flow can be easily interrupted manually at any time simply by kinking or applying pressure to the flexible tubing without damage to the apparatus.

Referring particularly to Fig. 4, the apparatus has at the free end of the resistance conduit 8 a male luer fitting 44 for fitment on the inlet end of the container 12 of Fig. 1 which has a complementary female fitting (not shown).

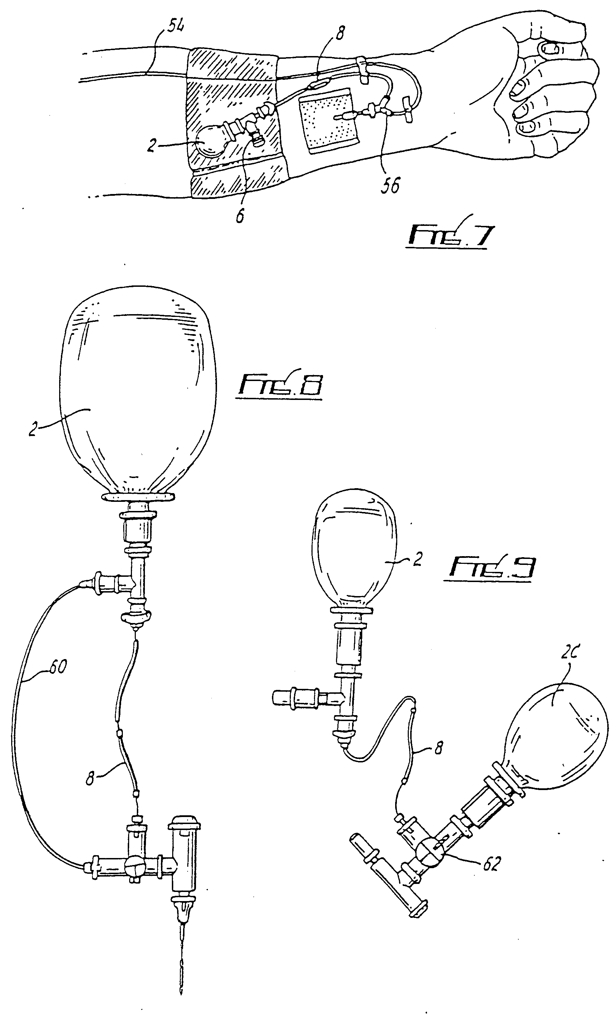

In Fig. 5, a saline reservoir 46 is located about one metre above the patient with a flexible tubing 48 leading the saline to the patient through an injection needle (not shown). A T-piece adaptor 50 is included in the tubing 48 and its branch leads through tubing 52 to a resistance conduit 8 and balloon 2 similar to those shown in Fig. 4. The balloon 2 is filled with 100 ml of a drug to be injected into the patient without interruption of the saline flow from the reservoir 46. The pressure of the drug within the balloon 2 is equal to 2 metres of water and the drug therefore is fed under pressure through the T-piece adaptor 50 into the tubing 48 and thence to the patient. Thus the drug is introduced without the requirement for nursing attention to switch the flow from saline to drug and back to saline.

The higher pressure in the balloon 2 than that provided by the reservoir 46 ensures that the balloon 2 does not reflate with saline after being emptied of drug. Thus the drug and saline supplies are connected in parallel rather than in series.

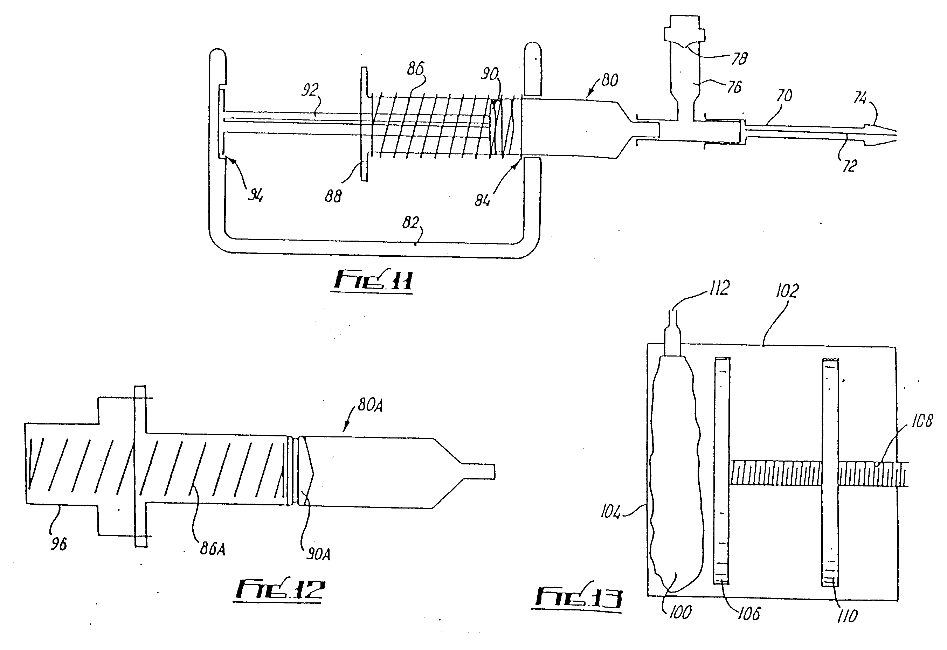

Fig. 6 shows how pressure of fluid in a balloon 2a can be maintained by connection with balloon 2b while feeding its fluid through a resistance conduit 8a to a patient. The balloon 26 leads through a T-piece adaptor 4b and a resistance conduit 8b to an inflatable member 52 located within the balloon 2a. The resistance conduits 8a and 8b are selected to allow the same rate of fluid flow through them, so as fast as fluid flows from the balloon 2a its volume is replaced in balloon 2a by inflation of the member 52 by fluid from the balloon 2b.

The balloon 2b is larger and inflated to a greater pressure than the balloon 2a to ensure a positive flow to the balloon 2a. Thus the operating pressure of balloon 2b is 400 mm Hg while that of the balloon 2a is 200 mm of Hg.

Fig. 7 illustrates the portable nature of an embodiment of the apparatus of the invention, with a main infusion line 54 terminating in a Y-piece adaptor 56 to which a balloon containing a drug is connected through a resistance conduit 8. The balloon 2 empties itself over a period of 20 minutes and is replenished as required through a one-way valve 6.

Figs. 8 and 9 show alternative apparatus of the invention for providing both a supply of drugs and a flushing facility. In Fig. 8 the flushing is provided by a branch line 60 which by-passes the resistance conduit 8, while in Fig. 9 the flushing is by way of a second balloon 2c downstream of the conduit 8 and isolated from it by a 3-way valve 62.

The storage system of Fig. 10 terminates in a valve 64 and provides a pressurised inflated balloon 2 containing a drug for fitment to a resistance conduit, thereby providing a rapid replacement for a depleted drug supply, or an alternative drug supply to one already being administered from a similar system, for example through an injection syringe.

The method can be applied for example to subcutaneous or intravascular injections.

Referring now to Fig. 11, a 20 cm length of flexible medical-grade polyvinyl chloride resistance tubing 70 of 4 mm diameter has a through bore 72 of 0.068 mm diameter and terminates at one end in a male luer lock fitting 74 for connection to a feed tube and hypodermic needle. At its opposite, inlet end the tubing 70 is connected to a valve housing 76 having a one-way valve 78 and thence to a syringe 80 which is mounted on a fixed holder 82. The holder 82 is apertured at 84 to allow sliding passage of the body of the syringe 80 and a compression spring 86 bears against a shoulder 88 at the end of the body and against the portion of the holder 82 surrounding the aperture 84. The syringe has a piston 90 to which is fixed a piston rod 92 which fits within a recess 94 at a rear portion of the holder 82. The spring exerts a force equivalent to a head of 200 mm of mercury.

In use, the syringe is charged with a drug or other fluid through the one-way valve 78, causing the body of the syringe 80 to move to the right through the aperture 84 until it contains the desired amount of fluid. The spring 86 then causes the body to move to the left through the aperture, resulting in the piston 90 pressurising the fluid and forcing it through the bore 72 of the resistance tubing 70. The length and diameter of the bore 72 dictate the rate of flow through it, and in this case the fluid passes at 0.2 ml per hour.

Fig. 12 shows an alternative arrangement for the syringe 80A. In this case the spring 86A is mounted within the body of the syringe to bear against the piston 90A. At its other end the spring 86A bears against a cap 96 fitted over the end of the syringe 80A. Thus the spring 86A pushes directly against the piston 90A to expel the fluid.

In Fig. 13 the reservoir is a flexible bag 100 disposed in a housing 102 between an end wall 104 and a movable plate in the form of a piston 106. The piston 106 is rotatably mounted on a screw-threaded rod 108 around which a coil spring 110 is located with the inner end of the spring 110 secured to the rod 108. The opposite end of the spring 110 is fixed to the housing 102.

The flexible bag 100 has an outlet 112 to which can be attached a flow line and flow restrictor as in the other embodiments.

In use, the coil spring 110 is tensioned and as it unwinds it rotates the rod 108, causing the piston 106 to move towards the bag 100 and compress it against the wall 104 of the housing 102, thus ejecting its fluid contents through the outlet 112.

By selecting suitable pressures from the reservoir and suitable lengths and diameters for the bore through the resistance tubing, flow rates down to 0.1 ml per hour can be obtained.

By providing a flexible resistance tube the fluid flow rate can be controlled within fine tolerances and the length of the tube can be increased to reduce the flow rate. Such increase of the tube length does not interfere with the fluid feed, as the flexible tube not only performs the function of flow rate control but also provides at least a portion of the flexible feed tubing to the patient. Thus the tube does not render the apparatus unwieldy or obtrusive when substantial lengths are required, allowing much slower feed rates to be obtained than hitherto.

Modifications and improvements may be made without departing from the scope of the invention.

## Claims

1. A method of manufacturing a flexible tube for use in fluid feed apparatus, comprising forming around a thin elongate member a body of flexible material so that the elongate member is embedded in said body, and withdrawing the elongate member longitudinally from said body thereby to form a bore through the body.

2. A method as claimed in Claim 1, wherein the elongate member is a wire.

3. A method as claimed in Claim 1 or 2, wherein the ratio of the diameter of the body of flexible material to the diameter of the elongate member is greater than 2:1.

4. A method as claimed in Claim 1, 2 or 3 wherein the diameter of the elongate member is between 0.05 and 0.25 mm.

5. A method as claimed in any one of the preceding Claims, wherein the body is formed around the elongate member by extrusion.

6. A method as claimed in any one of the preceding Claims, wherein the body is made of a material selected from silicone rubber, polyurethane and polyvinyl chloride.
